# EUROPÄISCHE PATENTSCHRIFT

(11) **EP 0 464 832 B1**
(45) Veröffentlichungstag und Bekanntmachung des Hinweises auf die Patenterteilung: **25.11.1998**
(21) Anmeldenummer: 91111155.7
(22) Anmeldetag: 04.07.1991
(51) Int. Cl.: C12N 15/55, C12N 9/86, C12Q 1/34, C12N 1/21

(54) **Klonierung der N-Methylhydantoinase**
Cloning of N-methylhydantoinase
Clonage du N-méthylhydantoinase

(30) Priorität: 06.07.1990 DE 4021571
(43) Veröffentlichungstag der Anmeldung: 08.01.1992
(73) Patentinhaber: BOEHRINGER MANNHEIM GMBH, 68298 Mannheim (DE)
(72) Erfinder: Schumacher, Günther, Dr., W-8139 Bernried (DE); Burtscher, Helmut, Dr., W-8121 Habach (DE); Möllering, Hans, W-8132 Tutzing (DE)
(74) Vertreter: Huber, Bernhard, Dipl.-Chem.

(56) Entgegenhaltungen:
- EP-A- 0 154 269
- EP-A- 0 219 034
- EP-A- 0 437 254
- WO-A-88/09373
- BIOLOGICAL ABSTRACTS; Band 86, nr.5; 1988 Zusammenfassung nr.45041; SIEDEL, J. et al.: "Fully enzymatic colorimetric assay of serum and urine creatinine wich obviates the need for sample blank measurements"

## Beschreibung

Das Enzym N-Methylhydantoinase (NMHase) wird zur Bestimmung des Creatiningehalts in Flüssigkeiten benötigt. Der Creatininspiegel ist ein wesentlicher Parameter für die Nierendiagnostik. Weltweit werden jährlich ca. eine Milliarde Tests durchgeführt. Daher ist eine kostengünstige Bereitstellung des Enzyms NMHase, sowie die Möglichkeit einer problemlosen Fermentation Grundvoraussetzung für die Bereitstellung von diagnostischen Kits zur Creatininbestimmung. Das Molekulargewicht der NMHase beträgt 125 kD im SDS-Gel. Die spezifische Aktivität beträgt 2 U/mg, die K_{M} für N-Methylhydantoin liegt bei 2x10⁻⁵ mol/l. Üblicherweise wird die NMHase aus Arthrobacter isoliert. Dieses Verfahren besitzt jedoch Nachteile aufgrund des verwendeten Mikroorganismus.

EP-A-0 154 269 beschreibt eine 1-Methylhydantoinase, welche 1-Methylhydantoin in Gegenwart eines Nukleosidtriphosphats und eines mehrwertigen Metallions hydrolysiert. Dieses Enzym wird durch Züchtung von Arthrobacter spec., Moraxella spec., Micrococcus spec. oder Brevibacterium spec. erhalten und kann als Reagenz zur Bestimmung von Creatinin eingesetzt werden.

Die Veröffentlichung Siedel et al. (Anal. Lett. 21 (6): 1009-1018, 1988) beschreibt einen enzymatischen kolorimetrischen Test zur Bestimmung von Creatinin in Serum und Urin unter Verwendung einer ATP-abhängigen l-Methylhydantoinase.

EP-A-0 437 254 beschreibt ein Verfahren und Reagenz zur enzymatischen Bestimmung von Creatinin, wobei das in der Probe enthaltene Creatinin mittels der Creatinin-Iminohydrolase zur 1-Methylhydantoin und zu einem ersten Molekül NH₃ umgesetzt, das gebildete 1-Methylhydantoin mittels der ATP-abhängigen 1-Methylhydantoinase und der Carbamoylsarcosin-Amidohydrolase in Sarcosin, CO₂ und ein zweites Molekül NH₃ überführt und beide gebildeten Moleküle NH₃ gemeinsam bestimmt werden.

Zur Bereitstellung größerer Mengen von NMHase müssen daher verbesserte Anreicherungsverfahren entwickelt werden. Dies war auch die Aufgabe der vorliegenden Erfindung.

Die erfindungsgemäße Aufgabe konnte durch Klonierung des die NMHase kodierenden Gens aus Arthrobacter und seine Expression in einem geeigneten Wirtsorganismus gelöst werden.

Ein Gegenstand der vorliegenden Erfindung ist somit eine isolierte DNA, welche die in SEQ ID NO: 1 dargestellte Nukleotidsequenz enthält und für ein Protein mit NMHase-Aktivität kodiert.

Ein weiterer Gegenstand der vorliegenden Erfindung ist ein rekombinanter Vektor, der eine oder mehrere Kopien einer DNA unter Kontrolle eines regulierbaren Promotors enthält, wobei die DNA (1) die in SEQ ID NO: 1 dargestellte Nukleinsäuresequenz, (2) eine ihr im Rahmen der Degeneration des genetischen Codes entsprechende Sequenz oder (3) eine mit einer Sequenz aus (1) oder/und (2) unter stringenten Bedingungen hybridisierende Sequenz besitzt und für ein Protein mit N-Methylhydantoinase-Aktivität kodiert.

Unter einer Hybridisierung unter stringenten Bedingungen im Sinne der vorliegenden Erfindung wird dabei auf Maniatis et al. (1982) "Molecular Cloning. A laboratory manual", Cold Spring Harbor Laboratory, New York, verwiesen.

Die erfindungsgemäße DNA kodiert für ein Protein mit 1288 Aminosäuren, dessen Sequenz in SEQ ID NO: 2 dargestellt ist. Die vorliegende Erfindung beinhaltet somit auch ein Protein mit NMHase-Aktivität und der in SEQ ID NO: 2 dargestellten oder einer davon abgeleiteten Aminosäuresequenz, das durch gentechnologische Verfahren gewonnen wird, z.B. durch Expression in einem heterologen Organismus, d.h. in einem Organismus, in dem das für das erfindungsgemäße Protein kodierende Gen ursprünglich nicht vorkommt. Andererseits ist es auch möglich, durch Einführung einer oder mehrerer Kopien der erfindungsgemäßen DNA in einem Organismus, in dem eine erfindungsgemäße DNA vorliegt, eine verbesserte Expression des NMHase-Gens zu erreichen.

Weiterhin ein Gegenstand der vorliegenden Erfindung ist ein rekombinanter Vektor, der eine oder mehrere Kopien einer erfindungsgemäßen DNA enthält. Ein erfindungsgemäßer rekombinanter Vektor kann ein Vektor sein, der zur Proteinexpression in prokaryontischen oder eukaryontischen Organismen geeignet ist. Vorzugsweise ist er ein prokaryontischer Vektor.

Ein erfindungsgemäßer rekombinanter Vektor kann ein Vektor sein, der in einer Wirtszelle extrachromosomal vorliegt (z.B. Plasmid) oder sich in das Genom des Wirts integriert (z.B. Bakteriophage Lambda). Vorzugsweise ist der erfindungsgemäße rekombinante Vektor ein Plasmid. Ein geeignetes erfindungsgemäßes Plasmid ist z.B. das Plasmid pBP010.

Auf einem erfindungsgemäßen rekombinanten Vektor befindet sich die DNA, die für ein Protein mit NMHase-Aktivität kodiert, unter Kontrolle eines regulierbaren Promotors, d.h. daß eine Expression der erfindungsgemäßen DNA z.B. durch einen Repressor unterdrückt werden kann und nur bei einer gezielten Induktion des regulierbaren Promotors stattfindet. Diese Induktion kann beispielsweise durch eine Temperaturänderung oder durch Zugabe eines chemischen Induktors (z. B. IPTG bei lac-Promotor-Derivaten) erfolgen. In einer besonders bevorzugten Ausführungsform der vorliegenden Erfindung handelt es sich bei dem regulierbaren Promotor, unter dessen Kontrolle sich das NMHase-Gen befinden soll, um den über Katabolit-Repression durch Zucker, wie z. B. Glucose und Fructose, regulierbaren mgl-Promotor aus Salmonella typhimurium (WO 88/09373).

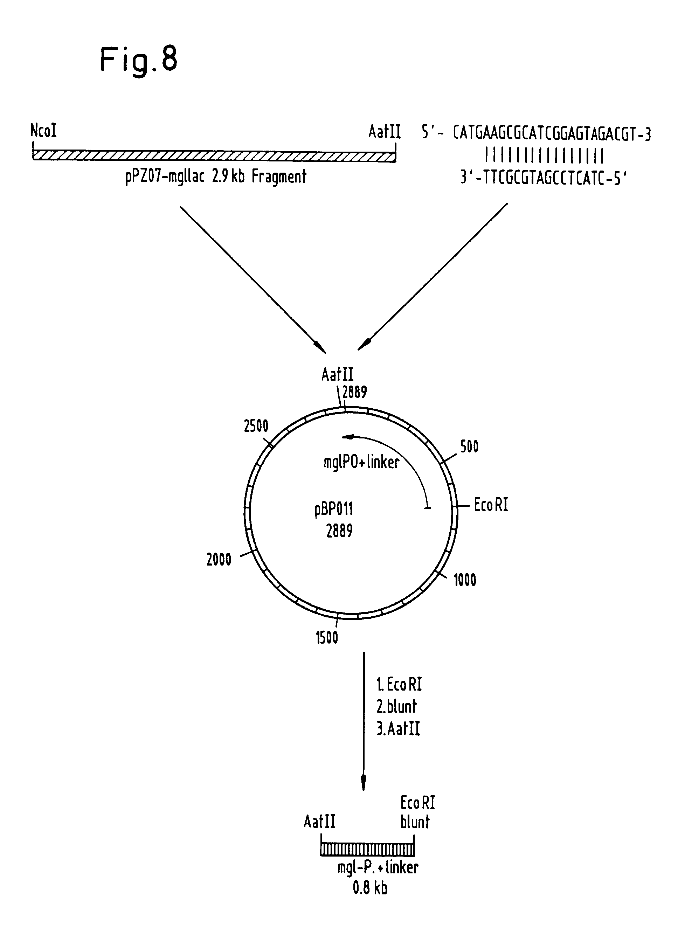

Ein geeigneter erfindungsgemäßer Vektor zur Expression von NMHase in gram-negativen Bakterien, insbesondere E.coli ist z. B. das Plasmid pB006. Zur Herstellung von pB006 wurde ein DNA-Fragment, welches die Sequenz des mgl-Promotors aus Salmonella typhimurium enthält, aus dem Plasmid pBZ07-mgl-lac (beschrieben in der W088/09373, Fig. 8) isoliert und vor ein DNA-Frament kloniert, welches die kodierende Sequenz des NMHase-Gens aus Arthrobacter ohne dessen eigenen Promotor enthält.

Weiterhin ein Gegenstand der vorliegenden Erfindung ist eine Zelle, die einen erfindungsgemäßen rekombinanten Vektor, auf dem sich die für ein Protein mit NMHase-Aktivität kodierende DNA unter Kontrolle eines tac- oder eines mgl-Promotors befindet, enthält. Vorzugsweise ist diese Zelle eine Bakterienzelle, besonders bevorzugt eine E.coli Zelle.

Die erfindungsgemäße DNA wurde durch Klonierung des NMHase-Gens gewonnen. Dazu wurde chromosomale DNA von Arthrobacter nach gängigen Methoden isoliert und mit geeigneten Restriktionsenzymen gespalten. Von diesen DNA-Fragmenten wurde eine Genbank in E.coli angelegt. Eine Klonierung des NMHase-Gens auf übliche Weise (Mustern der Genbank mit Oligonukleotidsonden und Selektion der Klone über NMHase-Aktivität) führte jedoch nicht zum Erfolg. Es wurde nämlich bei einer Klonierung in E.coli bei keinem der verwendeten Arthrobacter DNA-Fragmente eine NMHase-Aktivität festgestellt. Dieser Befund war überraschend, da aufgrund der Hybridisierung mit der Oligonukleotid-Sonde ein DNA-Fragment der richtigen Länge mit Start- und Stopcodon identifiziert werden konnte. Eine NMHase-Aktivität konnte erst bei Klonierung von DNA-Fragmenten nachgewiesen werden, auf denen der native NMHase-Promotor fehlte.

Weiterhin ein Gegenstand der Erfindung ist auch ein Verfahren zur Gewinnung eines Proteins mit NMHase-Aktivität, bei dem man eine Zelle mit einer erfindungsgemäßen DNA oder einem erfindungsgemäßen rekombinanten Vektor transformiert, die transformierten Zellen in einem geeigneten Medium kultiviert und das Protein aus dem Medium oder den Zellen anreichert.

Vorzugsweise verwendet man als Wirtsorganismus für das erfindungsgemäße Verfahren E. coli Bakterien. Dabei ist es jedoch günstig, daß eine Kultivierung der transformierten Zellen unter suboptimalen Wachstumsbedingungen stattfindet. Unter suboptimalen Wachstumsbedingungen ist etwa eine verringerte Temperatur bei der Inkubation (30°C oder weniger), eine Reduzierung des Sauerstoffeintrags oder/und die Verwendung eines Minimalmediums (d.h. eines Mediums, das gewisse für den kultivierten Organismus essentielle Nährstoffe in limitierender Konzentration enthält) zu verstehen.

So sind etwa die Anzuchtbedingungen bei einem Verfahren zur Gewinnung von NMHase aus E. coli, bei dem man einen rekombinanten Vektor verwendet, welcher das NMHase-Gen unter Kontrolle des tac-Promotors enthält, ein Minimalmedium, eine Inkubationstemperatur ≤ 30°C und eine unvollständige Induktion des tac-Promotors mit 0,8% Lactose.

Die besonders bevorzugte Expression des NMHase-Gens unter Kontrolle des mgl-Promotors von Salmonella typhimurium findet ebenfalls vorzugsweise bei einer Inkubationstemperatur von 30°C oder weniger, gegebenenfalls verbunden mit einer zusätzlichen Reduzierung des Sauerstoffeintrags statt, so daß die gebildete NMHase nicht in inaktiver Form als Präzipitationskörper anfällt. Der mgl-Promotor wird durch Katabolit-Repression reguliert (EP-A 0 316 378).

Allgemein ist es für das erfindungsgemäße Verfahren bevorzugt, daß die Induktion des jeweils verwendeten regulierbaren Promotors nur unvollständig durchgeführt wird, was ebenfalls zu einer verringerten Bildung von Präzipitationskörpern beiträgt.

Weiterhin ist es für das erfindungsgemäße Verfahren besonders bevorzugt, daß man zur Stabilisierung, vorzugsweise während der Anreicherung der NMHase aus den transformierten Zellen oder dem Medium das Protein mit dem Enzymsubstrat N-Methylhydantoin inkubiert. Überraschenderweise kann nämlich die Stabilität der durch das erfindungsgemäße Verfahren gewonnen rekombinanten NMHase, bei Anwesenheit einer Menge von ungefähr 3,8 nmol N-Methylhydantoin pro Einheit (U) des Enzyms, stark erhöht werden. Dazu wird das Enzym mit einer N-Methylhydantoin-Lösung, vorzugsweise in einer Konzentration von 1 bis 100 mmol/l, besonders bevorzugt 10 bis 70 mmol/l, am meisten bevorzugt 50 mmol/l, inkubiert. Bei diesem Inkubationsschritt ist es günstig, die Temperatur auf z. B. 55 °C zu erhöhen. Überraschend ist insbesondere, daß die Anwesenheit des eigenen Substrats das Enzym stabilisiert und aber gleichzeitig die enzymatische Reaktion des rekombinanten Enzyms nicht störend beeinflußt.

Das nach einem erfindungsgemäßen Verfahren erhaltene Protein kann neben den üblichen Bestandteilen in einem Reagenz zur Bestimmung des Creatiningehalts in Flüssigkeiten eingesetzt werden.

Die folgenden Beispiele sollen zusammen mit den Sequenzprotokollen und Figuren 1 bis 10 die Erfindung weiter verdeutlichen. Es zeigen:
- SEQ ID NO: 1: die DNA-Sequenz des NMHase-Gens,
- SEQ ID NO: 2: die daraus abgeleitete Aminosäuresequenz der NMHase,
- Fig. 1: ein 6 kb großes EcoRI-Fragment aus Arthrobacter mit einem ca. 0,6 kb großen Fragment des NMHase-Gens,
- Fig. 2: ein 3,7 kb großes SalI-Fragment aus Arthrobacter mit einem 3,0 kb großen, für das NMHase-Gen kodierenden Bereich,
- Fig. 3: den 3'-terminalen Bereich des NMHase-Gens,
- Fig. 4: einen EcoRI/AatII-Linker,
- Fig. 5: die Herstellung des Plasmids pBP008,
- Fig. 6: die Herstellung des Plasmids pBP009,
- Fig. 7: die Herstellung des NMHase-Expressions-Plasmids pBP010,
- Fig. 8: die Herstellung des Plasmids pBP011 mit dem mgl-Promotor aus Salmonella typhimurium,
- Fig. 9: die Gewinnung von Fragmenten des NMHase-Gens aus pBP010,
- Fig. 10: die Herstellung des NMHase-Expressions-Plasmids pBP006.

### Beispiel 1

### Klonierung der NMHase

Aus Arthrobacter spec. DSM 2563 wurde DNA nach gängigen Methoden isoliert (J. Marmur - A procedure for the isolation of deoxyribonucleic acid from micro-organisms, J.Mol.Biol. 3, 208-218 (1961); S. Visuvanathan et al. - Simple enzymic method for isolation of DNA from diverse bacteria, Journal of Microbiological Methods 10, 59-64 (1989)) und mit den Restriktionsenzymen EcoRI oder HindIII gespalten. Als Klonierungsvektor für die Arthrobacter-DNA wurde Bakteriophage λ gt10 verwendet (Boehringer Mannheim GmbH). Die Arthrobacter-DNA wurde gemäß Vorschrift des Herstellers in λ gt10 kloniert.

Die erhaltene Arthrobacter-Genbank wurde mit einer Oligonukleotid-Sonde gemustert, die aus einer Teilpeptid-Sequenz der NMHase abgeleitet wurde.

Teilpeptid-Sequenz der NMHase:

Met Lys Arg Ile Gly Val Asp Val Gly Gly Thr Phe Thr Asp Leu
Tyr Phe.

Aus dieser Teilpeptid-Sequenz wurden die folgenden Oligonukleotid-Sonden abgeleitet:

1. ATG AA(G/A) (C/A)G(G/A) AT(A/C/T) GG(G/A/T/C) GT
2. ATG AA(G/A) (C/A)G(T/C) AT(A/C/T) GG(G/A/T/C) GT
3. ATG AAG CGC ATC GGC GTG GAC GTG GGC GGC ACG TTC ACC
GAT CTG TAC TT

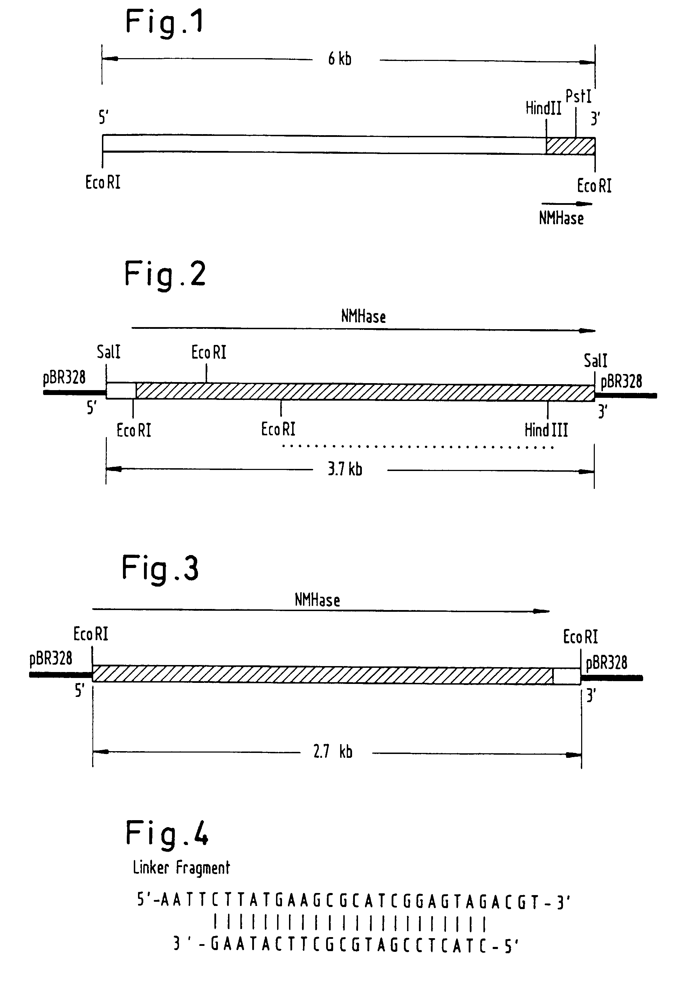

Mit Hilfe dieser Oligonukleotid-Sonden wurde ein 6 kb großes EcoRI-Fragment in der λ gt10-Genbank gefunden, das einen Teil des NMHase-Gens (ca. 0,6 kb) enthält (Fig. 1).

Ein Teil dieses Fragments (ca. 300 bp zwischen den PstI und EcoRI-Schnittstellen) wurde mit ³²P radioaktiv markiert. Anschließend wurde Arthrobacter DNA mit dem Restriktionsenzym SalI geschnitten, auf einem Agarose-Gel aufgetrennt und in einem Southern Blot mit dem radioaktiv markierten DNA-Fragment hybridisiert. Der hybridisierende DNA-Bereich wurde aus dem Agarose-Gel ausgeschnitten und in die SalI-Restriktions-Schnittstelle des Tetracyclin-Resistenzgens von pBR328 (Boehringer Mannheim GmbH) kloniert.

Eine Untersuchung von mit diesem Plasmid transformierten E. coli Zellen erbrachte ein DNA-Fragment von 3,7 kb Länge, welches einen 3,0 kb großen Bereich des NMHase-Gens enthält (Fig. 2).

Das durch eine gepunktete Linie gekennzeichnete EcoRI/ HindIII-Fragment aus dieser Insertion wurde mit Digoxigenin markiert (Boehringer Mannheim, Dig Kit). Mit dieser Sonde wurde wieder die bereits oben erwähnte λ gt10-Genbank gemustert, wobei ein 2,7 kb großes Stück gefunden wurde, das den 3'-terminalen Bereich des NMHase-Gens enthält (Fig. 1). Auch dieses DNA-Fragment wurde in den Vektor pBR328 kloniert.

### Beispiel 2

### Expression der NMHase

### 2.1 Übliche Verfahren

Das 3,7 kb große SalI-Fragment (Fig. 2) wurde in den kommerziell erhältlichen Vektor pUC19 (Boehringer Mannheim GmbH) kloniert. Anschließend wurde das NMHase-Gen durch Einklonierung des EcoRI-Fragments aus Fig. 3 vervollständigt. Ein derartiges Konstrukt führte jedoch nicht zur Expression aktiver NMHase.

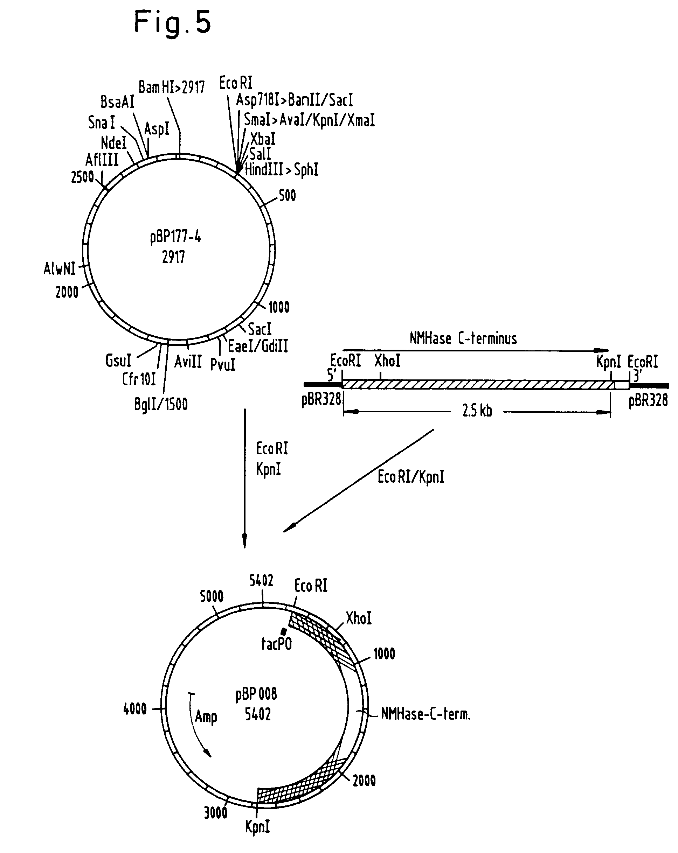

Auch ein Versuch, eine Expression dieses Konstrukts durch Klonierung unter Kontrolle eines induzierbaren tac-Promotors und Induzierung des tac-Promotors auf übliche Weise (Inkubation bei 37°C, Vollmedium und vollständige Induktion des tac-Promotors) zu erreichen, scheiterte. Dazu wurde das Plasmid pKK177-3 (DSM 3062) mit EcoRI und HindIII geschnitten und mit einem über EcoRI und HindIII aus pUC19 herausgeschnittenen Polylinker ligiert. Das entstehende Plasmid wurde pBP177-4 bezeichnet. Das Plasmid pBP177-4 wurde anschließend mit EcoRI und KpnI geschnitten und mit einem 2,5 kb großen C-terminalen NMHase-Fragment (ebenfalls mit EcoRI und KpnI geschnitten) zum Plasmid pBP008 vereinigt (Fig. 5).

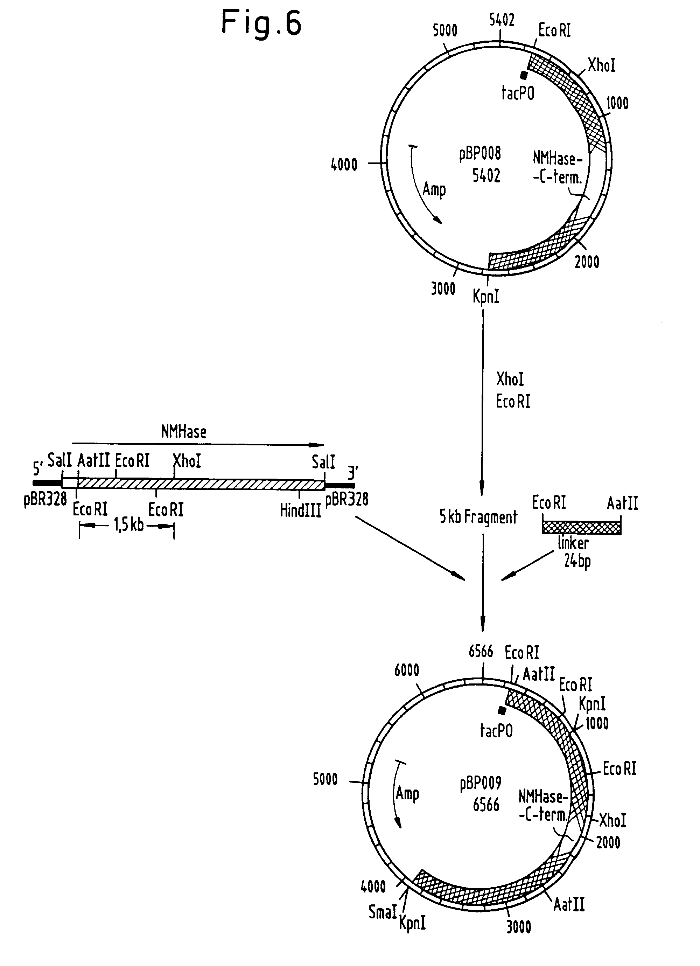

Das Plasmid pBP008 wurde mit den Enzymen XhoI und EcoRI geschnitten und das resultierende große (5kb) Fragment mit einem 1,5 kb Fragment aus dem NMHase-N-Terminus, welches die Endschnittstellen AatII und XhoI besitzt, und einem EcoRI-AatII-Linker (siehe Fig. 4) zu Plasmid pBP009 vereinigt (Fig. 6). In E. coli Zellen, die mit diesem Plasmid transformiert waren, wurde ein Protein, dessen Molekulargewicht in etwa dem der NMHase entsprach, exprimiert. Es war jedoch keine enzymatische Aktivität nachzuweisen.

### 2.2 Erfindungsgemäßes Verfahren

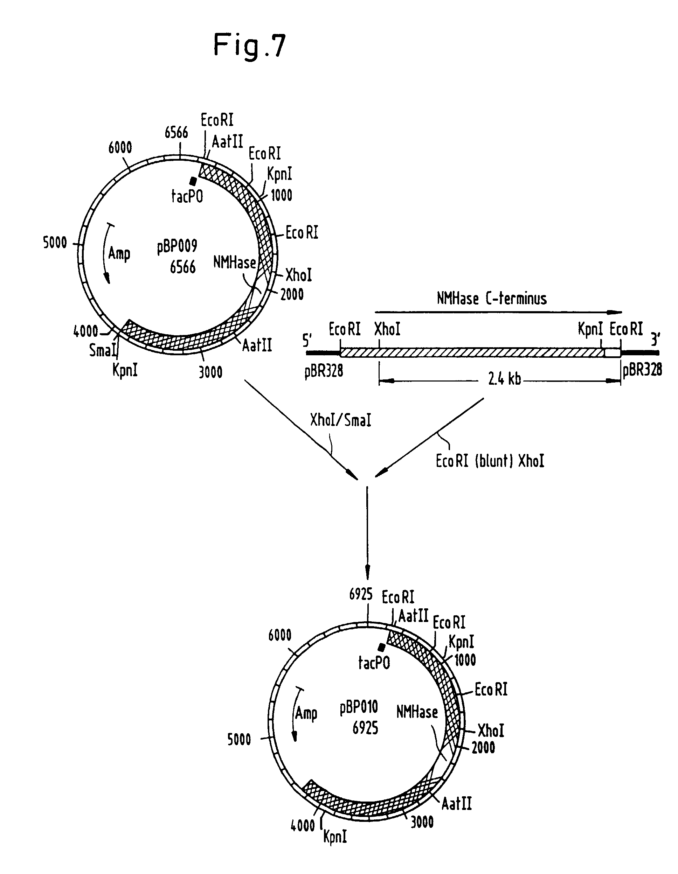

Zunächst erfolgte eine C-terminale Erweiterung der NMHase. Dazu wurde das Plasmid pBP009 (Fig. 6) mit den Enzymen XhoI und SmaI geschnitten und ein dabei resultierendes 5,5kb großes DNA-Fragment isoliert, welches den tac-Promotor, den N-terminalen Bereich der NMHase und das Ampicillin Resistenzgen enthält. Dieses Fragment wurde mit einem C-terminalen NMHase-Fragment aus pBR328, welches die Endschnitt-stellen EcoRI (glatte Enden durch Behandlung mit Klenow-Polymerase) und XhoI besitzt, zu Plasmid pBP010 vereinigt (Fig. 7). pBP010 ist in der Lage, NMHase zu exprimieren.

Im nächsten Schritt wurde das NMHase-Gen unter Kontrolle des mgl-Promotors aus Salmonella typhimurium (beschrieben in der W088/09373) gebracht. Dazu wurde das Plasmid pPZ07/mgllac (beschrieben in der W088/09373) mit den Enzymen NcoI und AatII geschnitten und daraus ein 2,9 kb großes DNA-Fragment isoliert, welches den mgl-Promotor enthält. Dieses Fragment wurde mit einem NcoI-AatII-Linker zu Plasmid pBP011 vereinigt (Fig. 8).

Das Plasmid pBP011 wurde mit EcoRI geschnitten, zur Erzeugung von glatten Enden mit Klenow-DNA-Polymerase behandelt und mit AatII nachgeschnitten. Anschließend wurde ein resultierendes 0,8 kb großes DNA-Fragment mit einem glatten und einem AatII-Ende, welches den mgl-Promotor und das Linker-Fragment enthält, isoliert (Fig. 8).

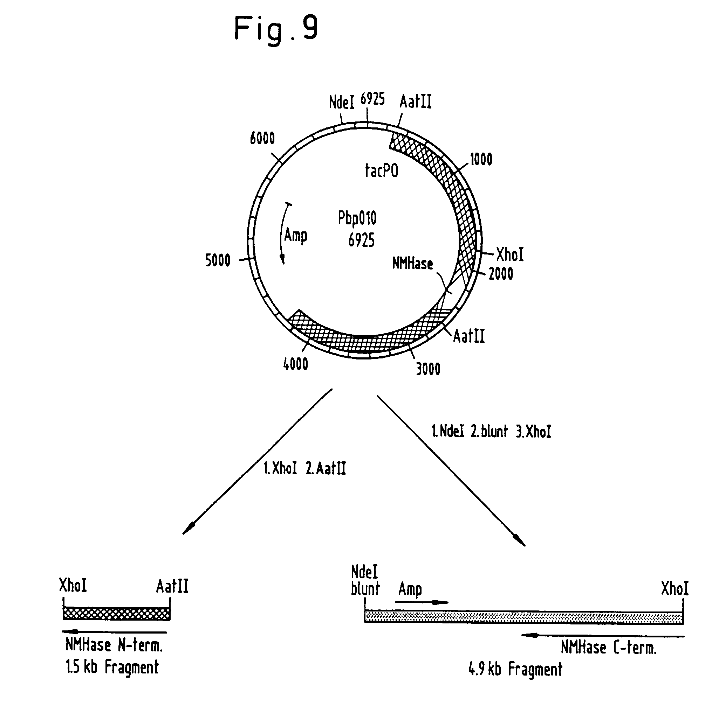

Das Plasmid pBP010 wurde mit NdeI geschnitten und zur Erzeugung von glatten Fragmentenden mit Klenow-DNA-Polymerase behandelt. Anschließend wurden diese Fragmente mit XhoI geschnitten und ein 4,9 kb grosses Fragment isoliert, welches den C-terminalen Bereich des NMHase-Gens enthält (Fig. 9).

Das Plasmid pBP010 wurde auch mit XhoI und AatII geschnitten, wobei ein 1,5 kb großes Fragment isoliert werden konnte, welches den N-terminalen Bereich des NMHase-Gens enthält (Fig. 9).

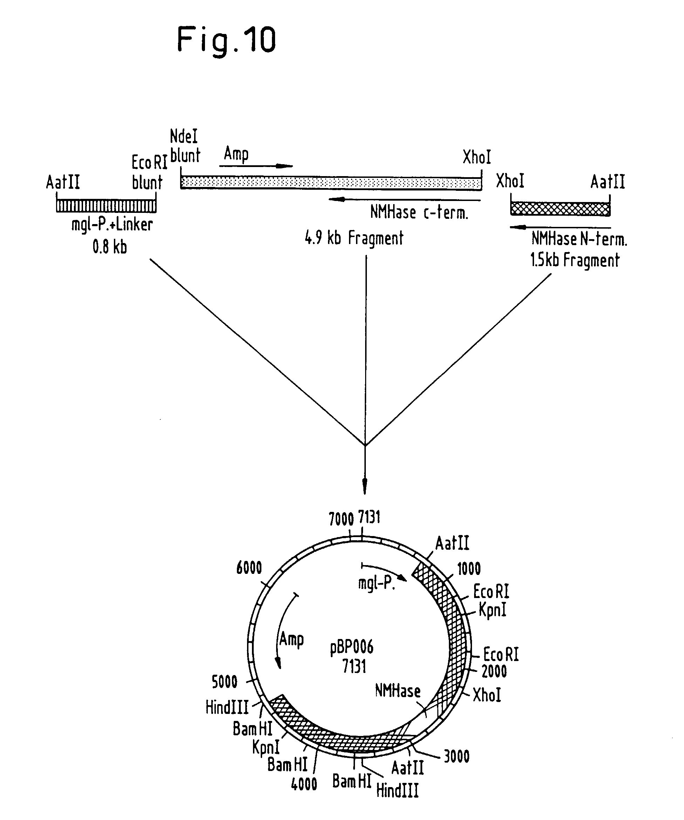

Die beiden Fragmente aus dem Plasmid pBP010 (4,9kb und 1,5kb) wurden mit dem 0,8 kb Fragment aus pBP011, das den mgl-Promotor enthält, ligiert. Das resultierende Plasmid wurde pBP006 bezeichnet (Fig. 10) und ist in der Lage, NMHase zu exprimieren.

### Beispiel 3

### Fermentation und Anreicherung rekombinanter NMHase in E. coli

E.coli HB101-Zellen (DSM 1607) wurden mit dem NMHase Expressionsplasmid pBP010 transformiert. Um eine bessere Regulierbarkeit des tac-Promotors zu gewährleisten, wurden die Zellen zusätzlich mit einem zu pBP010 kompatiblen Plasmid transformiert, das das lacI^{q}-Gen enthält.

Das lacI^{q}-Gen ist dem Fachmann schon lange bekannt und leicht erhältlich. Als kompatible Plasmide zu pBP010 kommt z.B. pACYC 177 (DSM 3693P) oder davon abgeleitete in Frage.

### 3.1 Anzucht und Vorkultur

2x500 ml LB-Medium mit Kanamycin und Ampicillin in zwei 2000 ml Erlenmeyerkolben wurden mit E. coli
HB101/lacI^{q}/pBP010-Zellen beimpft. Dann wurde 16 Stunden bei 37°C und 150 Upm (Rundschüttler, Braun Certomat M) inkubiert. Die OD bei 578 nm betrug in der 10. Stunde ca. 3,0 bis 4,0, bei einem pH von ca. 7,6.

### Hauptfermentation:

| Nährboden und Hauptkultur: | |
|---|---|
| Glycerin 86% | 2500 g |
| Lactose | 500 g |
| NH₄Cl | 50 g |
| MgSO₄ * 7 H₂O | 50 g |
| K₂HPO₄ | 150 g |
| Caseinpepton | 3000 g |
| Ammoniaklösung 25% Merck 5432 | 500 ml |
| Wasser | 100 l |

### Fermentationsverlauf:

Nach dem Beimpfen (1% Inokulum) geht die Kultur ohne Verzögerung in das exponentielle Wachstum über. Die Temperatur des Fermenters wird bis zur OD578nm von 1,400 bei 28°C gehalten. Bei Erreichen der gewünschten OD wird die Temperatur auf 25°C zurückgenommen, das Wachstum verlangsamt sich. Weiterhin kann der Sauerstoffeintrag reduziert werden. Diese Maßnahmen sind notwendig, um das Wachstum zu limitieren und damit die Bildung von Präzipitationskörpern (Inclusion Bodies) entgegen zu wirken. Wichtig ist der richtige Zeitpunkt des Temperaturshifts, wird er zu früh durchgeführt, verzögert sich das Wachstum um Stunden, wird er zu spät durchgeführt, erhält man nur unlösliches Protein.

Durch die zusätzliche Reduzierung des Sauerstoffs erreicht man eine weitere Steigerung der Aktivität. Bei der Fermentation mit Temperatur-Shift liegt die Ausbeute bei ca. 2500 U/L, max. 3000 U/L (150 U/OD) nach 30 Stunden. Mit der zusätzlichen Reduzierung der 02 -Menge im Medium werden nach 45 Stunden bis 4000 U/L erreicht.

### Beispiel 4

### Gewinnung von rekombinanter NMHase aus E. coli

### 4.1 Messung der Enzymaktivität

Die Bestimmung der Enzymaktivität erfolgt mittels eines Farbtests, der Carbamoyl-Sarcosin-Hydrolase, Sarcosin-Oxidase, Peroxidase, N-Methylhydantoin, 4-Aminoantipyrin, Tribrom-3-hydroxybenzoesäure, ATP und MgCl₂ in Phosphatpuffer, pH 7,8 enthält.

### Meßprinzip:

NMHase wandelt das zugesetzte N-Methylhydantoin in Carbamoyl-Sarcosin um, Carbamoyl-Sarcosin-Hydrolase wandelt dieses in Sarcosin um, dieses wird durch Sarcosin-Oxidase abgebaut zu Glycin, Formaldehyd und Wasserstoffperoxid. Die Peroxidase wandelt mit Hilfe des entstandenen Wasserstoffperoxids die zugesetzten Farbsubstrate in einen dunkel-violetten Farbstoff um. Gemessen wird die Extinktions-Zunahme bei einer Wellenlänge von 546nm. Der Enzymtest ist ausführlich in der EP-A 0 154 269 beschrieben.

Eine Einheit (U) ist definiert als µmol Carbamoyl-Sarcosin-Bildung pro Minute bei 25°C unter Meßbedingungen im gekoppelten Test mit Carbamoyl-Sarcosin-Hydrolase, Sarcosin-Oxidase und Peroxidase. Bei einer 5 ml Testkultur wird eine Aktivität von 0,16 U/ml erhalten. Dies entspricht einer Steigerung gegenüber der Ausgangskultur (Arthrobacter spec. DSM 2563) um einen Faktor von ca. 20.

### 4.2 Enzymreinigung

315 g Biomasse (gemäß Beispiel 3), resultierend aus 10 1 Fermentationskultur mit einer Gesamtaktivität von 16 KU NMHase, wurde mit 2 1 0,1 mol/l Kaliumphosphat-Puffer, enthaltend 10 % Glycerin, pH 8,0, suspendiert und durch Behandlung mit Lysozym und 1 mal 700 bar Hochdruck-Dispersion aufgeschlossen. Zur Negativ-Abtrennung der Nucleinsäuren und Zelltrümmer erfolgte der Zusatz einer 10 %igen Polyethylenimin-Lösung G20 (Luvalgan, MG 20 000), bis kein weiterer Niederschlag mehr auftrat und alle NMHase-Aktivität im Überstand verblieb. Hierzu wurden bei Raumtemperatur 3 Vol-% G 20-Lösung zugesetzt, 30 min gerührt und danach zentrifugiert. Zum NMHase-Überstand wurden 8 Vol-% feucht abgepreßtes DEAE-Sephadex im batch zugegeben und nach 2 Stunden Rühren waren 95 % des Enzyms adsorbiert. Nach Filtration wurde der Austauscher mit Phosphatpuffer gewaschen und die NMHase mit 0,5 mol/l Ammoniumsulfat-Lösung, enthaltend 0,1 mol/l K-PO₄-Puffer, pH 8,0, eluiert. Das Eluat hatte eine spezifische Aktivität von 1,1 U/mg Protein. Anschließend erfolgte eine 10 minütige Erwärmung bei 55°C in Gegenwart von 50 mmol/l N-Methylhydantoin (Endkonzentration), wobei störende Fremdproteine ausgefällt wurden. Nach Zentrifugation wurde der klare Überstand mit Ammoniumsulfat auf 2,2 mol/l weitergesättigt und die dabei ausgefallene NMHase abzentrifugiert. Es schließen sich 2 Kristallisationen an, die erste Kristallisation bei einer Protein-Konzentration von ca. 60 mg/ml, pH 8,0, 0,1 mol/l K-PO4-Puffer, 1,27 mol/l Ammoniumsulfat. Es bilden sich nach kurzer Zeit Prismen. Die Kristallisation war nach 24 Std. beendet, im abzentrifugierten Überstand waren nur noch 5 % NMHase. Die NMHase-Kristalle wurden in 0,1 mol/l K-PO₄-Puffer gelöst und nach Abtrennung vom Ungelösten wurde die Enzymlösung einer zweiten Kristallisation unterworfen (1,05 mol/l Ammoniumsulfat-Konzentration). Die über Nacht aufgetretenen Enzym-Kristalle wurden abgetrennt, mit Puffer aufgenommen, gegen 20 mmol/l Phosphatpuffer dialysiert, mit 2 Teilen Raffinose versetzt (bezogen auf Protein Menge) und lyophilisiert.
Die Ausbeute betrug 5,8 KU NMHase = 34 % der Ausgangsaktivität mit einer spezifischen Aktivität von 2,15 U/mg Protein.

Nach jedem Anreicherungsschritt wurde die Enzymaktivität gemäß Beispiel 4.1 getestet.

Die Fremdaktivitäten Katalase, Creatinase, Creatininase und Carbamoyl-Sarcosin-Hydrolase waren null. Es wurde eine minimale Fremdoxidase-Aktivität (= Summe aus Glucose-Oxidase, Pyruvat-Oxidase, Lactat-Oxidase, Uricase und Cholesterin-Oxidase) von 0,002 % festgestellt.

Die Eigenschaften der rekombinanten NMHase bezüglich pH-Optimum, pH-Stabilität, Temperaturabhängigkeit, thermische Stabilität, K_{M}, ATP- und Magnesiumabhängigkeit, Ammoniumabhängigkeit und Molekulargewicht entsprachen den Eigenschaften der NMHase aus Arthrobacter.

### Beispiel 5

### Sequenzierung des NMHase-Gens

Fragmente aus dem NMHase kodierenden Gen wurden in den Klonierungsvektor M13 subkloniert und nach Standard-Techniken sequenziert. Die Nukleinsäuresequenz ist in SEQ ID NO: 1 dargestellt. Daraus resultiert ein Protein mit 1288 Aminosäuren, dessen Sequenz in SEQ ID NO: 2 angegeben ist.
SEQ ID NO:1
   SEQUENZLÄNGE:3867 Basenpaare
   ART DER SEQUENZ:Nukleotidsequenz 
ATGAAGCGCA TCGGAGTAGA CGTCGGCGGC ACCTTCACCG ACTTGTATTT TTCGGACGAT 60
GACCAGCGCA TCGCTGTGGT CGAGAAGGTT CCCTCGACTC CTCACGACCC GTCCGAGGCC 120
GTGATCAATG GCATTAAGAA GCTCTGTGAG AAGGCGGGAG TGTCTCTGTC AGAGATCGAC 180
CAGCTGGTCC ATGGGACTAC GGTAGCCACC AACACCGCAC TAACGCACAC TGGCGCGGAA 240
GTCGGGATGA TTACTACCGA GGGCTTCCGG GATATCTTGC ATATCGCCAG GCACAAAAAA 300
CCGCATAATT TCTCTCTGCA GCAGGATCTG CCGTGGCAGA CCAAACCACT GATCAAGCGC 360
CGGTATCGGC TCACCGTTAA GGAACGTATC ACCGCGCCGC ACGGTGAGAT CCTGGTCCCT 420
TTGGATGAGG ATGAGGTCCG ACAGAGAGTG CGTGAGCTCA AGACAGCTGG CGTGCAGGCC 480
ATCGCTGTAT GTCTGTTGCA TTCGTATTTG AACCCGGAGC ACGAGCAGCG AATCGGCGAG 540
ATCGTCAATG AGGAATTCCC CGAGGCGTAT CTTTCCCTGT CTTCTGAAAT TGTGCCTCTA 600
TATCGAGAGT ATGAACGATT CTCAACTACC GCATTAAATG CCTACGTTGG CCCTAGGGTC 660
TCGCGCTACC TGCATCGCCT GCAGGAGCAG GCCGAAAATT TGGGGTACCA GCGCGAAATC 720
CTGCTAATGC AGTCTTCAGG CGGCATGGTG CCTATTGGTG AAGCTGCGAA ACGGCCGGTG 780
ACGTTGATGA TGTCCGGTCC AGTGGGAGGT CTGATCGGTG GTATGTGGGC TGCTAAGCAG 840
TCTGGATTTG AGAACGTGGT TACCCTAGAT ATCGGGGGCA CCTCTGCGGA TATCGGCGTT 900
GCCTACCAGG GTGAGTTGCG CATGCGCCAC CTGCTGGACA CGAAGATCGG TGATCATCAA 960
GCCATGGTTC CCATGGTGGA TATCGACACT ATCGGTGCCG GCGGCGGTTC GATCGCCTAT 1020
GTTGATGCTG GTGGCGTCTT CCGCGTGGGC CCCCAGTCAG CTGGTGCTGT TCCGGGGCCG 1080
GTCTGTTACG GCCGCGGTGG AACGGAACCA ACGTCAACCG ATGCTCAGGT ACTGCTCGGA 1140
AGGATGCGTC CAGACAGAAT TCTGGCCGGC TCGGGTTTGG ACATGGATCT CGACCGTGCC 1200
CGCGCTGCCA TGCAAGGACT GGCCGACAAG CTCGGCATGT CCATCGAAGA AGCGGCACTG 1260
GGTGCGCTTC AGATCCAGAA GTTTGGAATG ACCCAGGCCA TTGAGCAGAA CTCAGTTCGC 1320
CGGGGGTATG ATCCGCGAGA TTTCACTCTT GTCGCTGCCG GTGGAGCTGG CGCCTTGTTC 1380
GCCTGTGAGA TTGCTGCTGA ACTCGAAGTG CCGCACGTAC TGGTCCCGGC TCATCCAGGC 1440
ATCATCGCAG GTATCGGGTT GCTGGCCACG GATGAGCAAT ACGAGTTTGT GGCAACCAAC 1500
CGGTTCAGCT TTGCTTTCCG TGACGCTGCG GTCATCCAAG CGTCCTACGA GCAGCTCGAG 1560
CGCGAACGTA ACGCTCAACT GGATGCCGAA GAAGTCCCCG CCGAACGGCG CAAAATTGTT 1620
TGGCTGCGTG ACGCTCGATA TGAGGGCCAA GGCTATGAGA TCCGCTTCGT CGTACCCGAG 1680
GGGCCGGTCA CTACCGCATG GTTGGACCAA GCAGAAGCCG CTTTCCACGA TGCCCACTTC 1740
GAGGAATACG GCCACCGCTT TAAGGGCGGC ACCGTAGAGG TGATCAATAT CAGGGTGGAA 1800
GCCCGTGCCG TTATGGATGA ACTGCCCACG CCAGAAGCGA CGCAGTCAGG CTCACTTGAA 1860
AATGCGTTGG TGGAGACCCG CCCTGTAACT TTCCAGCAAG CAGGTAAGCC TGTCACCTTG 1920
GACACCGGAT TCTACGACCG GGCCAAGATG GGAATCGGAA CCACGTTCGC CGGACCGGTG 1980
GTCATCGAGC AGTACGACTC CACCACAGTG ATTCCTCCAG GTTTCACCGG GACGGTGGAT 2040
GATGCCGGCA ACCTGGTCAT CGCTTGCCCA GCGGTCACCC AGACTGTGGA GAAGCTGGCC 2100
ACCCCGATTC TCATGCGCGT CATCGGCGGC GCGTTGAACT CGGCGGCCAA AGAAATGGCT 2160
TCGGTGCTTT TCCGCATGTC TTACTCATCG ATCATCCGCG AATCGGAGGA TCTGGGAGCT 2220
GGCCTCTTCG ATAAGGACGG AAACGTCCTG GCCGAATCAG ATTCCACCCC AATGTTCATG 2280
GGCTCCATGC CGAAAATTGT CAAAGGTGTC ATCTCTGTCC TGGGCGACGA CATCCATGAT 2340
GGCGACGTCA TCTTGCACAA TGATCCGTAC TTGGGGGCTA CGCACTCCCC GGATGTTGCA 2400
ATCATCGAAC CCATCTTCCA CGATGGAGAA CTCGTCGGTT TCGCTGGAGC CTCCGGGCAA 2460
CTGATCGATA ACGGTGGCGC ATTTTCTGGA CTGATGGTAG ATATTCAGGA CGTGCAGTCC 2520
GAAGGAACCA TCTTCCGGGC GGTGAAGGTC TATGAGAAGG GTGTTCGTCA GGAGTCACTG 2580
ATCCGGCACA TCCTGAACAA CACTCGCACA CCTACCTCTA ACGAGGGCGA CTTCCAGGCA 2640
ATGATCGCCG CGTGTGATCT GGCCAAGTCC CGTTACTTGG CCCTGGTCGA GCGGTATGGC 2700
CGAGACTCGG TTCGTGACGC CGGGCAGTTC TGGATCGATT ATTCAGAGCG TATCGTACGC 2760
CAGGAAATCG CTAAGATTCC GGATGGTGTG TACGAAACCG AGACAGGCTA CTTGGACGAT 2820
GACGGACGCA ACTACGGCAA AAAGCTTCCC ATCGTCGTGA AGGTCATTGT TGAGGGCGAT 2880
GAGATTACCT ACGACCTCAC AGGATCCTCC GCACAGGTGC CGACGGCCTA CAACTGCGCA 2940
 
TTCGAAGGAA CCACTGTCTC GGCGTTCACG TTCATCACCC GCATGATGTT CTTGGATGAG 3000
GTCGCGTTCC CGGTATTCGT CCCACAGAAC GAGGGCATGC TCAAAGCGTT GAAGGTGATC 3060
GCACCGAAGG GAACTATCTT CAATCCGAAC TACCCGGCGG CTACTTTTAG CAGATTCTCC 3120
CAGGTGCAGC GTGCCGTCGA CCTAGCGTTG CGAGCGCTGG CCCCGGTCAT GCCCGAACGA 3180
GTTACTGCCG GAAACTCGGC CCATATCCAC TTCATGTCCT ACTCTGGCTG GGACGAAAAG 3240
CAAGGTGAGT ACTGGGTCTA TCTGGAAGTC AATGAGGGTT CCTATGGAGC TCGCCAGGAC 3300
TCCGACGGCC CAGATTCGGT TGACAACCTC ATCGCCAACA CCCGCAATAA TCCGATCGAA 3360
GAACTCGAAT GGCGGTTCCC GATGCGTACT GACCGCTACG AGCTACGCGA GGATCCGGCC 3420
GCCGCCGGCG AATACCGTGG CGGAATCGGC ATTGTCCGGG AGAACACCTT CTTGGAGGAT 3480
ACTGCGGTGA CCTGCGAGGG CGAACGTCAC GATTCAGATG TCCCATGGGG CGCCTATGGC 3540
GGCCACGACG GTCTGAATGC GTCCCTGATA AAGAACCCAG GCCGCGACGG GGAAGAGTCC 3600
TGGCCGTCAA AGGTCACCGG TCGTCAGTTG CAAGCCGGTG ATTCCTTGCA GATCACGGTA 3660
CCTAGCGGCG GTGGTTTCGG AGACCCGCTC AAGCGCAACC CATTGCAGGT TCTCGAAGAT 3720
GTGCTCGATG GATTCACCAC CACCGAAGCC GCTTCCAGGG ACTACGGTGT GATTCTCAAA 3780
ACGGTCAATG GTCAACTCAC CGTCGATCTA GCGGCCACCG CTGTAAAACG GGAGAACGCA 3840
GTCTCTGAGC TCAGCCACAC CAACTGA 3867
SEQ ID NO:2
   SEQUENZLÄNGE:1288 Aminosäuren
   ART DER SEQUENZ: Aminosäuresequenz

Met Lys Arg Ile Gly Val Asp Val Gly Gly Thr Phe Thr Asp Leu Tyr
1               5                   10                  15
Phe Ser Asp Asp Asp Gln Arg Ile Ala Val Val Glu Lys Val Pro Ser
            20                  25                  30
Thr Pro His Asp Pro Ser Glu Ala Val Ile Asn Gly Ile Lys Lys Leu
        35                  40                  45
Cys Glu Lys Ala Gly Val Ser Leu Ser Glu Ile Asp Gln Leu Val His
    50                  55                  60
Gly Thr Thr Val Ala Thr Asn Thr Ala Leu Thr His Thr Gly Ala Glu
65                  70                  75                  80
Val Gly Met Ile Thr Thr Glu Gly Phe Arg Asp Ile Leu His Ile Ala
                85                  90                  95
Arg His Lys Lys Pro His Asn Phe Ser Leu Gln Gln Asp Leu Pro Trp
            100                 105                 110
Gln Thr Lys Pro Leu Ile Lys Arg Arg Tyr Arg Leu Thr Val Lys Glu
        115                 120                 125
Arg Ile Thr Ala Pro His Gly Glu Ile Leu Val Pro Leu Asp Glu Asp
    130                 135                 140
Glu Val Arg Gln Arg Val Arg Glu Leu Lys Thr Ala Gly Val Gln Ala
145                 150                 155                 160
Ile Ala Val Cys Leu Leu His Ser Tyr Leu Asn Pro Glu His Glu Gln
                165                 170                 175
Arg Ile Gly Glu Ile Val Asn Glu Glu Phe Pro Glu Ala Tyr Leu Ser
            180                 185                 190
Leu Ser Ser Glu Ile Val Pro Leu Tyr Arg Glu Tyr Glu Arg Phe Ser
        195                 200                 205
Thr Thr Ala Leu Asn Ala Tyr Val Gly Pro Arg Val Ser Arg Tyr Leu
    210                 215                 220
His Arg Leu Gln Glu Gln Ala Glu Asn Leu Gly Tyr Gln Arg Glu Ile
225                 230                 235                 240
Leu Leu Met Gln Ser Ser Gly Gly Met Val Pro Ile Gly Glu Ala Ala
                245                 250                 255
Lys Arg Pro Val Thr Leu Met Met Ser Gly Pro Val Gly Gly Leu Ile
            260                 265                 270
Gly Gly Met Trp Ala Ala Lys Gln Ser Gly Phe Glu Asn Val Val Thr
        275                 280                 285
Leu Asp Ile Gly Gly Thr Ser Ala Asp Ile Gly Val Ala Tyr Gln Gly
    290                 295                 300
Glu Leu Arg Met Arg His Leu Leu Asp Thr Lys Ile Gly Asp His Gln
305                 310                 315                 320
Ala Met Val Pro Met Val Asp Ile Asp Thr Ile Gly Ala Gly Gly Gly
                325                 330                 335
Ser Ile Ala Tyr Val Asp Ala Gly Gly Val Phe Arg Val Gly Pro Gln
            340                 345                 350
Ser Ala Gly Ala Val Pro Gly Pro Val Cys Tyr Gly Arg Gly Gly Thr
        355                 360                 365
Glu Pro Thr Ser Thr Asp Ala Gln Val Leu Leu Gly Arg Met Arg Pro
    370                 375                 380
Asp Arg Ile Leu Ala Gly Ser Gly Leu Asp Met Asp Leu Asp Arg Ala
385                 390                 395                 400

Arg Ala Ala Met Gln Gly Leu Ala Asp Lys Leu Gly Met Ser Ile Glu
                405                 410                 415
Glu Ala Ala Leu Gly Ala Leu Gln Ile Gln Lys Phe Gly Met Thr Gln
            420                 425                 430
Ala Ile Glu Gln Asn Ser Val Arg Arg Gly Tyr Asp Pro Arg Asp Phe
        435                 440                 445
Thr Leu Val Ala Ala Gly Gly Ala Gly Ala Leu Phe Ala Cys Glu Ile
    450                 455                 460
Ala Ala Glu Leu Glu Val Pro His Val Leu Val Pro Ala His Pro Gly
465                 470                 475                 480
Ile Ile Ala Gly Ile Gly Leu Leu Ala Thr Asp Glu Gln Tyr Glu Phe
                485                 490                 495
Val Ala Thr Asn Arg Phe Ser Phe Ala Phe Arg Asp Ala Ala Val Ile
            500                 505                 510
Gln Ala Ser Tyr Glu Gln Leu Glu Arg Glu Arg Asn Ala Gln Leu Asp
        515                 520                 525
Ala Glu Glu Val Pro Ala Glu Arg Arg Lys Ile Val Trp Leu Arg Asp
    530                 535                 540
Ala Arg Tyr Glu Gly Gln Gly Tyr Glu Ile Arg Phe Val Val Pro Glu
545                 550                 555                 560
Gly Pro Val Thr Thr Ala Trp Leu Asp Gln Ala Glu Ala Ala Phe His
                565                 570                 575
Asp Ala His Phe Glu Glu Tyr Gly His Arg Phe Lys Gly Gly Thr Val
            580                 585                 590
Glu Val Ile Asn Ile Arg Val Glu Ala Arg Ala Val Met Asp Glu Leu
        595                 600                 605
Pro Thr Pro Glu Ala Thr Gln Ser Gly Ser Leu Glu Asn Ala Leu Val
    610                 615                 620
Glu Thr Arg Pro Val Thr Phe Gln Gln Ala Gly Lys Pro Val Thr Leu
625                 630                 635                 640
Asp Thr Gly Phe Tyr Asp Arg Ala Lys Met Gly Ile Gly Thr Thr Phe
                645                 650                 655
Ala Gly Pro Val Val Ile Glu Gln Tyr Asp Ser Thr Thr Val Ile Pro
            660                 665                 670
Pro Gly Phe Thr Gly Thr Val Asp Asp Ala Gly Asn Leu Val Ile Ala
        675                 680                 685
Cys Pro Ala Val Thr Gln Thr Val Glu Lys Leu Ala Thr Pro Ile Leu
    690                 695                 700
Met Arg Val Ile Gly Gly Ala Leu Asn Ser Ala Ala Lys Glu Met Ala
705                 710                 715                 720
Ser Val Leu Phe Arg Met Ser Tyr Ser Ser Ile Ile Arg Glu Ser Glu
                725                 730                 735
Asp Leu Gly Ala Gly Leu Phe Asp Lys Asp Gly Asn Val Leu Ala Glu
            740                 745                 750
Ser Asp Ser Thr Pro Met Phe Met Gly Ser Met Pro Lys Ile Val Lys
        755                 760                 765
Gly Val Ile Ser Val Leu Gly Asp Asp Ile His Asp Gly Asp Val Ile
    770                 775                 780
Leu His Asn Asp Pro Tyr Leu Gly Ala Thr His Ser Pro Asp Val Ala
785                 790                 795                 800
Ile Ile Glu Pro Ile Phe His Asp Gly Glu Leu Val Gly Phe Ala Gly
                805                 810                 815

Ala Ser Gly Gln Leu Ile Asp Asn Gly Gly Ala Phe Ser Gly Leu Met
            820                 825                 830
Val Asp Ile Gln Asp Val Gln Ser Glu Gly Thr Ile Phe Arg Ala Val
        835                 840                 845
Lys Val Tyr Glu Lys Gly Val Arg Gln Glu Ser Leu Ile Arg His Ile
    850                 855                 860
Leu Asn Asn Thr Arg Thr Pro Thr Ser Asn Glu Gly Asp Phe Gln Ala
865                 870                 875                 880
Met Ile Ala Ala Cys Asp Leu Ala Lys Ser Arg Tyr Leu Ala Leu Val
                885                 890                 895
Glu Arg Tyr Gly Arg Asp Ser Val Arg Asp Ala Gly Gln Phe Trp Ile
            900                 905                 910
Asp Tyr Ser Glu Arg Ile Val Arg Gln Glu Ile Ala Lys Ile Pro Asp
        915                 920                 925
Gly Val Tyr Glu Thr Glu Thr Gly Tyr Leu Asp Asp Asp Gly Arg Asn
    930                 935                 940
Tyr Gly Lys Lys Leu Pro Ile Val Val Lys Val Ile Val Glu Gly Asp
945                 950                 955                 960
Glu Ile Thr Tyr Asp Leu Thr Gly Ser Ser Ala Gln Val Pro Thr Ala
                965                 970                 975
Tyr Asn Cys Ala Phe Glu Gly Thr Thr Val Ser Ala Phe Thr Phe Ile
            980                 985                 990
Thr Arg Met Met Phe Leu Asp Glu Val Ala Phe Pro Val Phe Val Pro
        995                 1000                1005
Gln Asn Glu Gly Met Leu Lys Ala Leu Lys Val Ile Ala Pro Lys Gly
    1010                1015                1020
Thr Ile Phe Asn Pro Asn Tyr Pro Ala Ala Thr Phe Ser Arg Phe Ser
1025                1030                1035                1040
Gln Val Gln Arg Ala Val Asp Leu Ala Leu Arg Ala Leu Ala Pro Val
                1045                1050                1055
Met Pro Glu Arg Val Thr Ala Gly Asn Ser Ala His Ile His Phe Met
            1060                1065                1070
Ser Tyr Ser Gly Trp Asp Glu Lys Gln Gly Glu Tyr Trp Val Tyr Leu
        1075                1080                1085
Glu Val Asn Glu Gly Ser Tyr Gly Ala Arg Gln Asp Ser Asp Gly Pro
    1090                1095                1100
Asp Ser Val Asp Asn Leu Ile Ala Asn Thr Arg Asn Asn Pro Ile Glu
1105                1110                1115                1120
Glu Leu Glu Trp Arg Phe Pro Met Arg Thr Asp Arg Tyr Glu Leu Arg
                1125                1130                1135
Glu Asp Pro Ala Ala Ala Gly Glu Tyr Arg Gly Gly Ile Gly Ile Val
            1140                1145                1150
Arg Glu Asn Thr Phe Leu Glu Asp Thr Ala Val Thr Cys Glu Gly Glu
        1155                1160                1165
Arg His Asp Ser Asp Val Pro Trp Gly Ala Tyr Gly Gly His Asp Gly
    1170                1175                1180
Leu Asn Ala Ser Leu Ile Lys Asn Pro Gly Arg Asp Gly Glu Glu Ser
1185                1190                1195                1200
Trp Pro Ser Lys Val Thr Gly Arg Gln Leu Gln Ala Gly Asp Ser Leu
                1205                1210                1215
Gln Ile Thr Val Pro Ser Gly Gly Gly Phe Gly Asp Pro Leu Lys Arg
            1220                1225                1230

Asn Pro Leu Gln Val Leu Glu Asp Val Leu Asp Gly Phe Thr Thr Thr
        1235                1240                1245
Glu Ala Ala Ser Arg Asp Tyr Gly Val Ile Leu Lys Thr Val Asn Gly
    1250                1255                1260
Gln Leu Thr Val Asp Leu Ala Ala Thr Ala Val Lys Arg Glu Asn Ala
1265                1270                1275                1280
Val Ser Glu Leu Ser His Thr Asn
                1285

## Patentansprüche (Patentansprüche für folgende(n) Vertragsstaat(en): AT, BE, CH, DE, DK, FR, GB, GR, IT, LI, LU, NL, SE)

1. Isolierte DNA, **dadurch gekennzeichnet,**
daß sie die in SEQ ID NO. 1 dargestellte Nukleinsäuresequenz besitzt und für ein Protein mit N-Methylhydantoinase-Aktivität kodiert.

2. Rekombinanter Vektor,
dadurch gekennzeichnet, daß er eine oder mehrere Kopien einer DNA unter Kontrolle eines regulierbaren Promotors enthält, wobei die DNA (1) die in SEQ ID NO: 1 dargestellte Nukleinsäuresequenz, (2) eine ihr im Rahmen der Degeneration des genetischen Codes entsprechende Sequenz oder (3) eine mit einer Sequenz aus (1) oder/und (2) unter stringenten Bedingungen hybridisierende Sequenz besitzt und für ein Protein mit N-Methylhydantoinase-Aktivität kodiert.

3. Vektor nach Anspruch 2,
**dadurch gekennzeichnet,**
daß er eine DNA nach Anspruch 1 unter Kontrolle eines tac-Promotors enthält.

4. Vektor nach Anspruch 2,
**dadurch gekennzeichnet,**
daß er eine DNA nach Anspruch 1 unter Kontrolle eines mgl-Promotors enthält.

5. Zelle,
**dadurch gekennzeichnet,**
daß sie einen Vektor nach Anspruch 3 oder Anspruch 4 enthält.

6. Verfahren zur Gewinnung eines Proteins mit NMHase-Aktivität,
**dadurch gekennzeichnet,**
daß man
(1) eine Zelle mit einer DNA nach Anspruch 1 oder einem Vektor nach Anspruch 2 transformiert,
(2) die transformierten Zellen in einem geeigneten Medium kultiviert und
(3) das Protein aus dem Medium oder den Zellen anreichert.

7. Verfahren nach Anspruch 6,
**dadurch gekennzeichnet,**
daß man der NMHase zur Stabilisierung ungefähr 3,8 nmol N-Methylhydantoin pro Einheit (U) NMHase zusetzt.

8. Verfahren nach Anspruch 7,
**dadurch gekennzeichnet,**
daß man NMHase bei erhöhter Temperatur mit einer N-Methylhydantoin-Lösung inkubiert.

## Patentansprüche (Patentansprüche für folgende(n) Vertragsstaat(en): ES)

1. Verfahren zur Herstellung einer isolierten DNA, die die in SEQ ID NO. 1 dargestellte Nukleinsäuresequenz besitzt und für ein Protein mit N-Methylhydantoinase-Aktivität kodiert,
**dadurch gekennzeichnet,**
daß man von chromosomaler Arthrobacter DNA eine Genbank in E.coli anlegt und ein DNA-Fragment kloniert, auf dem der native NMH-ase Promotor fehlt.

2. Verfahren zur Herstellung eines rekombinanten Vektors,
**dadurch gekennzeichnet,**
daß man eine oder mehrere Kopien einer DNA in einem Vektor inseriert, so daß sie unter Kontrolle eines regulierbaren Promotors ist, wobei die DNA (1) die in SEQ ID NO. 1 dargestellte Nukleinsäuresequenz, (2) eine ihr im Rahmen der Degeneration des genetischen Codes entsprechende Sequenz oder (3) eine mit einer Sequenz aus (1) oder/und (2) unter stringenten Bedingungen hybridisierende Sequenz besitzt und für ein Protein mit N-Methylhydantoinase-Aktivität kodiert.

3. Verfahren nach Anspruch 2,
**dadurch gekennzeichnet,**
daß der Vektor eine DNA nach Anspruch 1 unter Kontrolle eines tac-Promotors enthält.

4. Verfahren nach Anspruch 2,
**dadurch gekennzeichnet,**
daß der Vektor eine DNA nach Anspruch 1 unter Kontrolle eines mgl-Promotors enthält.

5. Zelle,
**dadurch gekennzeichnet,**
daß sie einen Vektor mit einer oder mehreren Kopien einer DNA unter Kontrolle eines tac- oder mgl-Promotors enthält, wobei die DNA (1) die in SEQ ID NO. 1 dargestellte Nukleinsäuresequenz, (2) eine ihr im Rahmen der Degeneration des genetischen Codes entsprechende Sequenz oder (3) eine mit einer Sequenz aus (1) oder/und (2) unter stringenten Bedingungen hybridisierende Sequenz besitzt und für ein Protein mit N-Methylhydantoinase-Aktivität kodiert.

6. Verfahren zur Gewinnung eines Proteins mit NMHase-Aktivität,
**dadurch gekennzeichnet,**
daß man
(1) eine Zelle mit einer DNA, die die in SEQ ID NO 1 dargestellte Nukleinsäure besitzt und für ein Protein mit N-Methylhydantoinase-Aktivität kodiert, oder einem Vektor, der eine oder mehrere Kopien einer DNA unter Kontrolle eines regulierbaren Promotors enthält, wobei die DNA (1) die in SEQ ID NO. 1 dargestellte Nukleinsäuresequenz, (2) eine ihr im Rahmen der Degeneration des genetischen Codes entsprechende Sequenz oder (3) eine mit einer Sequenz aus (1) oder/und (2) unter stringenten Bedingungen hybridisierende Sequenz besitzt und für ein Protein mit N-Methylhydantoinase-Aktivität kodiert, transformiert,
(2) die transformierten Zellen in einem geeigneten Medium kultiviert und
(3) das Protein aus dem Medium oder den Zellen anreichert.

7. Verfahren nach Anspruch 6,
**dadurch gekennzeichnet,**
daß man der NMHase zur Stabilisierung ungefähr 3,8 nmol N-Methylhydantoin pro Einheit (U) NMHase zusetzt.

8. Verfahren nach Anspruch 7,
**dadurch gekennzeichnet,**
daß man NMHase bei erhöhter Temperatur mit einer N-Methylhydantoin-Lösung inkubiert.

## Claims (Claims for the following Contracting State(s): AT, BE, CH, DE, DK, FR, GB, GR, IT, LI, LU, NL, SE)

1. Isolated DNA,
wherein
it has the nucleic acid sequence shown in SEQ ID NO. 1 and codes for a protein with N-methylhydantoinase activity.

2. Recombinant vector
wherein
it contains one or several copies of a DNA under the control of a regulatable promoter, wherein the DNA has (1) the nucleic acid sequence shown in SEQ ID NO: 1, (2) a sequence corresponding to it within the scope of the degeneracy of the genetic code or (3) has a sequence which hybridizes with a sequence from (1) or/and (2) under stringent conditions and codes for a protein with N-methylhydantoinase activity.

3. Vector as claimed in claim 2,
wherein
it contains a DNA as claimed in claim 1 under the control of a tac promoter.

4. Vector as claimed in claim 2,
wherein
it contains a DNA as claimed in claim 1 under the control of a mgl promoter.

5. Cell,
wherein
it contains a vector as claimed in claim 3 or claim 4.

6. Process for the production of a protein with NMHase activity,
wherein
(1) a cell is transformed with a DNA as claimed in claim 1 or with a vector as claimed in claim 2,
(2) the transformed cells are cultured in a suitable medium and
(3) the protein is isolated from the medium or the cells.

7. Process as claimed in claim 6,
wherein
approximately 3.8 nmol N-methylhydantoin per unit (U) NMHase is added to the NMHase for stabilization.

8. Process as claimed in claim 7,
wherein
the NMHase is incubated with an N-methylhydantoin solution at an increased temperature.

## Claims (Claims for the following Contracting State(s): ES)

1. Process for the production of an isolated DNA, which has the nucleic acid sequence shown in SEQ ID NO. 1 and codes for a protein with N-methylhydantoinase activity
**wherein**
a gene bank is set up in E. coli from chromosomal Arthrobacter DNA and a DNA fragment is cloned on which the native NMH-ase promoter is missing.

2. Process for the production of a recombinant vector
**wherein**
one or several copies of a DNA are inserted into a vector so that it is under the control of a regulatable promoter, wherein the DNA has (1) the nucleic acid sequence shown in SEQ ID NO: 1, (2) a sequence corresponding to it within the scope of the degeneracy of the genetic code or (3) a sequence which hybridizes with a sequence from (1) or/and (2) under stringent conditions and codes for a protein with N-methylhydantoinase activity.

3. Process as claimed in claim 2,
**wherein**
the vector contains a DNA as claimed in claim 1 under the control of a tac promoter.

4. Process as claimed in claim 2,
**wherein**
the vector contains a DNA as claimed in claim 1 under the control of a mgl promoter.

5. Cell,
**wherein**
it contains a vector having one or several copies of a DNA under the control of a tac or mgl promoter, wherein the DNA has (1) the nucleic acid sequence shown in SEQ ID NO. 1, (2) a sequence corresponding to it within the scope of the degeneracy of the genetic code or (3) a sequence which hybridizes with a sequence from (1) or/and (2) under stringent conditions and codes for a protein with N-methylhydantoinase activity.

6. Process for the isolation of a protein with NMHase activity,
**wherein**
(1) a cell is transformed with a DNA which has the nucleic acid sequence shown in SEQ ID NO. 1 and codes for a protein with N-methylhydantoinase activity or with a vector that contains one or several copies of a DNA under the control of a regulatable promoter wherein the DNA has (1) the nucleic acid sequence shown in SEQ ID NO. 1, (2) a sequence corresponding to it within the scope of the degeneracy of the genetic code or (3) a sequence which hybridizes with a sequence from (1) or/and (2) under stringent conditions and codes for a protein with N-methylhydantoinase activity,
(2) the transformed cells are cultured in a suitable medium and
(3) the protein is isolated from the medium or the cells.

7. Process as claimed in claim 6,
**wherein**
approximately 3.8 nmol N-methylhydantoin per unit (U) NMHase is added to the NMHase for stabilization.

8. Process as claimed in claim 7,
**wherein**
the NMHase is incubated with an N-methylhydantoin solution at an increased temperature.

## Revendications (Revendications pour l'(les) Etat(s) contractant(s) suivant(s): AT, BE, CH, DE, DK, FR, GB, GR, IT, LI, LU, NL, SE)

1. ADN isolé caractérisé en ce qu'il possède la séquence d'acide nucléique représentée dans SEQ ID NO.1 et code une protéine à activité de N-méthylhydantoïnase.

2. Vecteur recombiné caractérisé en ce qu'il contient une ou plusieurs copies d'un ADN sous le contrôle d'un promoteur régulable, l'ADN (1) possédant la séquence d'acide nucléique représentée dans SEQ ID NO.1, (2) une séquence qui lui correspond dans le cadre de la dégénérescence du code génétique ou (3) une séquence qui s'hybride avec une séquence de (1) et/ou de (2) dans des conditions strictes et codant une protéine à activité de N-méthylhydantoïnase.

3. Vecteur selon la revendication 2 caractérisé en ce qu'il contient un ADN selon la revendication 1 sous le contrôle d'un promoteur tac.

4. Vecteur selon la revendication 2 caractérisé en ce qu'il contient un ADN selon la revendication 1 sous le contrôle d'un promoteur mgl.

5. Cellule caractérisée en ce qu'elle contient un vecteur selon la revendication 3 ou la revendication 4.

6. Procédé pour obtenir une protéine à activité de NMHase caractérisé en ce que
(1) on transforme une cellule avec un ADN selon la revendication 1 ou avec un vecteur selon la revendication 2,
(2) on cultive les cellules transformées dans un milieu approprié et
(3) on concentre la protéine à partir du milieu ou des cellules.

7. Procédé selon la revendication 6 caractérisé en ce que l'on ajoute à la NMHase pour la stabilisation environ 3,8. nmoles de N-méthylhydantoïne par unité (U) de NMHase.

8. Procédé selon la revendication 7 caractérisé en ce que l'on incube la NMHase à température augmentée avec une solution de N-méthylhydantoïne.

## Revendications (Revendications pour l'(les) Etat(s) contractant(s) suivant(s): ES)

1. Procédé de préparation d'un ADN isolé qui possède la séquence d'acide nucléique représentée dans SEQ ID NO.1 et code une protéine à activité de N-méthylhydantoïnase, caractérisé en ce que l'on constitue dans E. coli une banque génomique de l'ADN chromosomique d'Arthrobacter et on clone un fragment d'ADN sur lequel le promoteur natif de la NMHase est manquant.

2. Procédé de préparation d'un vecteur reconibiné caractérisé en ce que l'on insère une ou plusieurs copies d'un ADN dans un vecteur de sorte qu'il soit sous le contrôle d'un promoteur régulable, l'ADN (1) possédant la séquence d'acide nucléique représentée dans SEQ ID NO.1, (2) une séquence qui lui correspond dans le cadre de la dégénérescence du code génétique ou (3) une séquence qui s'hybride avec une séquence de (1) et/ou de (2) dans des conditions strictes et codant une protéine à activité de N-méthylhydantoïnase.

3. Procédé selon la revendication 2 caractérisé en ce que le vecteur contient un ADN selon la revendication 1 sous le contrôle d'un promoteur tac.

4. Procédé selon la revendication 2 caractérisé en ce que le vecteur contient un ADN selon la revendication 1 sous le contrôle d'un promoteur mgl.

5. Cellule caractérisée en ce qu'elle contient un vecteur préparé selon la revendication 3 ou la revendication 4.

6. Procédé pour obtenir une protéine à activité de NMHase caractérisé en ce que
(1) on transforme une cellule avec un ADN selon la revendication 1 ou avec un vecteur selon la revendication 2,
(2) on cultive les cellules transformées dans un milieu approprié et
(3) on concentre la protéine à partir du milieu ou des cellules.

7. Procédé selon la revendication 6 caractérisé en ce que l'on ajoute à la NMHase pour la stabilisation environ 3,8 nmoles de N-métbylhydantoïne par unité (U) de NMHase.

8. Procédé selon la revendication 7 caractérisé en ce que l'on incube la NMHase à température augmentée avec une solution de N-méthylhydantoïne.
